# EUROPÄISCHE PATENTSCHRIFT

(11) **EP 1 685 053 B1**
(45) Veröffentlichungstag und Bekanntmachung des Hinweises auf die Patenterteilung: **07.03.2007**
(21) Anmeldenummer: 04765270.6
(22) Anmeldetag: 16.09.2004
(51) Int. Cl.: B65H 63/06, D01H 13/22, D02G 1/16, G01B 11/10, G01N 21/89, G01N 33/36

(54) **VERFAHREN ZUR BESTIMMUNG VON EFFEKTEN EINES EFFEKTGARNES**
METHOD FOR DETERMINING THE EFFECTS OF A YARN
PROCEDE POUR DETERMINER DES EFFETS D'UN FILE

(30) Priorität: 16.10.2003 DE 10348742
(43) Veröffentlichungstag der Anmeldung: 02.08.2006
(73) Patentinhaber: Saurer GmbH & Co. KG, 41069 Mönchengladbach (DE)
(72) Erfinder: BIERMANN, Iris, 41239 Mönchengladbach (DE)
(74) Vertreter: Hamann, Arndt
(86) Internationale Anmeldenummer: PCT/EP2004/010368
(87) Internationale Veröffentlichungsnummer: WO 2005/037699

(56) Entgegenhaltungen:
- DE-A1- 10 026 389
- US-A- 3 303 698
- PATENT ABSTRACTS OF JAPAN Bd. 014, Nr. 531 (C-0780), 21. November 1990 (1990-11-21) -& JP 02 221427 A (MURATA MACH LTD), 4. September 1990 (1990-09-04)
- PATENT ABSTRACTS OF JAPAN Bd. 018, Nr. 430 (C-1236), 11. August 1994 (1994-08-11) & JP 06 128821 A (MURATA MACH LTD), 10. Mai 1994 (1994-05-10)

## Beschreibung

Die Erfindung betrifft ein Verfahren gemäß dem Oberbegriff des Anspruchs 1.

Bei der Herstellung von Garn wird üblicherweise eine möglichst hohe Gleichmäßigkeit des Garns in engen Toleranzen angestrebt. Für Effektgarne ist dagegen die Ungleichmäßigkeit des Garns charakteristisch. Als Effektgarn wird ein Garn bezeichnet, in dem Dickstellen mit vorgegebenen größeren Durchmessern und mit vorgegebenen Längen, die sogenannten Effekte, vorhanden sind. Die dazwischen liegenden Garnabschnitte mit geringerem Durchmesser werden als Stege bezeichnet.

Es ist bekannt, bei Meßbeginn an einer Spinnstelle über die ersten Garnmeter eine Durchmesser-Mittelwertbestimmung vorzunehmen. Dieser so genannte Referenzdurchmesser ist der Bezugsdurchmesser für alle weiteren Auswertungen. Bei einem Effektgarn würde ein derartig ermittelter Referenzdurchmesser aufgrund des Vorliegens von Effekten, also von Dickstellen, dicker angezeigt, als die Dicke des Steges tatsächlich ist. Die Erkennung der Ausbildung von Effekten ist auf dieser Basis einer einfachen Mittelwertbildung allein nur unzureichend möglich.

Die DE 100 26 389 A1 beschreibt eine Vorrichtung zur Überwachung eines laufenden Fadens mittels einer Sensoreinrichtung eines Spinnaggregates. Überschreitet der Wert des Durchmessers ausgewählte Tolerantgrenzen über eine vorbestimmte Länge, wird auf den Beginn einer Fehlerstelle im Faden geschlossen. Bewegt sich der Wert des Durchmessers anschließend wieder entsprechend lange innerhalb der Toleranzzone, wird auf das Ende der Fehlerstelle geschlossen. Dabei wird jede dieser Toleranzüberschreitungen als Garnfehler eingestuft. Die am laufenden Faden aufeinander folgend detektierten Durchmesserwerte werden als Kurvenverlauf über die Garnlänge erfasst und die Kurve in einem Datenspeicher hinterlegt. Der Datenspeicher enthält vorgegebene Kurvenverlaufsmuster, die einen Abschnitt des Kurvenverlaufs im Bereich einer Fehlerstelle repräsentieren, als Mustertypen. Diese Mustertypen gestatten aufgrund der Ausbildung ihres Kurvenverlaufes Rückschlüsse auf die Fehlerursachen. Um festzustellen, ob ein vorgegebener Mustertyp sich im Kurvenverlauf wiederholt, wird der Kurvenverlauf mit den vorgegebenen Mustertypen verglichen. Wird dabei im Kurvenverlauf erkannt, dass eine Fehlerstelle einem Mustertyp entspricht, wird anhand des erkannten Mustertyps Art des Fehlers und Fehlerursache ermittelt und deren Behebung ausgelöst.

Es ist Aufgabe der Erfindung, die Bestimmung von Effekten eines Effektgarnes zu verbessern.

Diese Aufgabe wird mit einem Verfahren mit den Merkmalen des Anspruchs 1 gelöst.

Vorteilhafte Ausgestaltungen der Erfindung sind Gegenstand der abhängigen Ansprüche.

Das erfindungsgemäße Verfahren ermöglicht es, die Effekte besser zu erkennen und den Effektdurchmesser, auch als Effektdicke bezeichnet, sowie die Effektlänge zutreffender zu ermitteln.

Der Stegdurchmesser, auch als Stegdicke bezeichnet, kann mit dem Verfahren nach Anspruch 3 weitgehend unbeeinflusst von den Effekten und somit wesentlich genauer ermittelt werden als es mit der bekannten einfachen Referenzwertbildung bei Garnmessungen möglich ist. Diese erhöhte Genauigkeit wirkt sich auch in der Genauigkeit der Effekterfassung positiv aus.

Mit einem Verfahren gemäß Anspruch 4 kann vermieden werden, dass ein nur sehr kurzes Über- oder Unterschreiten des Grenzdurchmessers zu einer Verfälschung der Effektlänge führt.

Vorteilhaft wird ein Variationswert bestimmt, der die Variation des Durchmessers auf der Effektlänge angibt. Dazu wird der Durchmesser innerhalb der Effektlänge fortlaufend gemessen. Der Variationswert kann als mittlere quadratische Ungleichmäßigkeit angegeben werden und gibt Auskunft über die Gleichmäßigkeit des Effektverlaufes. Aus dem Variationswert lassen sich Rückschlüsse auf die Qualität des späteren Endproduktes, beispielsweise eines-Gewebes, ziehen. Eine hohe Gleichmäßigkeit lässt ein sauberes Bild der Effekte im Gewebe erwarten, eine geringere Gleichmäßigkeit dagegen ein verwischtes Bild. Die Ermittlung der mittleren quadratischen Ungleichmäßigkeit entspricht der bekannten Ermittlung des so genannten CV-Wertes bei glattem Garn.

Das erfindungsgemäße Verfahren ermöglicht eine Erfassung der Effektlänge und Effektdicke mit Werten, die der wirklichen Ausbildung sehr nahe kommen, und damit zuverlässigere Aussagen über die Qualität des Effektgarnes und des Endproduktes.

Weitere Einzelheiten der Erfindung sind den Figuren entnehmbar.

Es zeigen:
- Fig. 1: eine Vorrichtung zur Durchführung des erfindungsgemäßen Verfahrens,
- Fig. 2: ein Effektgarn, dass durch die Aneinanderreihung von Messwerten des Garndurchmessers dargestellt ist,
- Fig. 3: die Prinzipdarstellung eines Garneffektes.

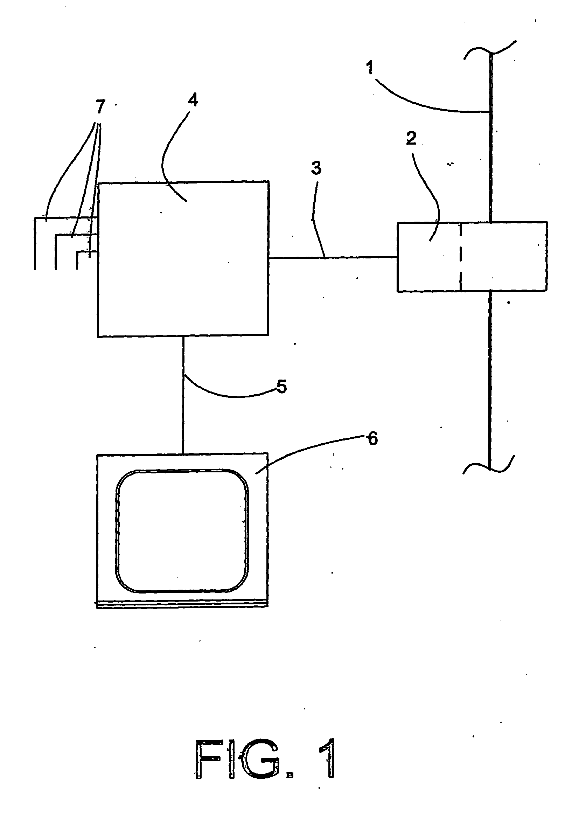

Fig. 1 zeigt einen Abschnitt eines Effektgarns 1, das einen Sensor 2 durchläuft, der zur Messung des Garndurchmessers D eingerichtet ist. Der Sensor 2 ist ein optischer Sensor, wie er im Prinzip bekannt ist und der daher hier nicht ausführlich erläutert wird. Der Sensor 2 ist über eine Leitung 3 mit der Auswerteeinheit 4 verbunden. Die Auswerteeinheit 4 ermittelt die gewünschten Effektdaten aus den vom Sensor 2 übermittelten Messwerten des Garndurchmessers D. Die Auswerteeinheit 4 überträgt die Effektdaten über die Leitung 5 an eine einen Monitor 6 umfassende Ausgabeeinrichtung. Auf dem Monitor 6 können die Effektdaten in gewünschter Form dargestellt werden.

Über die Leitungen 7 ist die Auswerteeinheit 4 mit weiteren nicht dargestellten Auswerteeinheiten oder Rechnern verbunden.

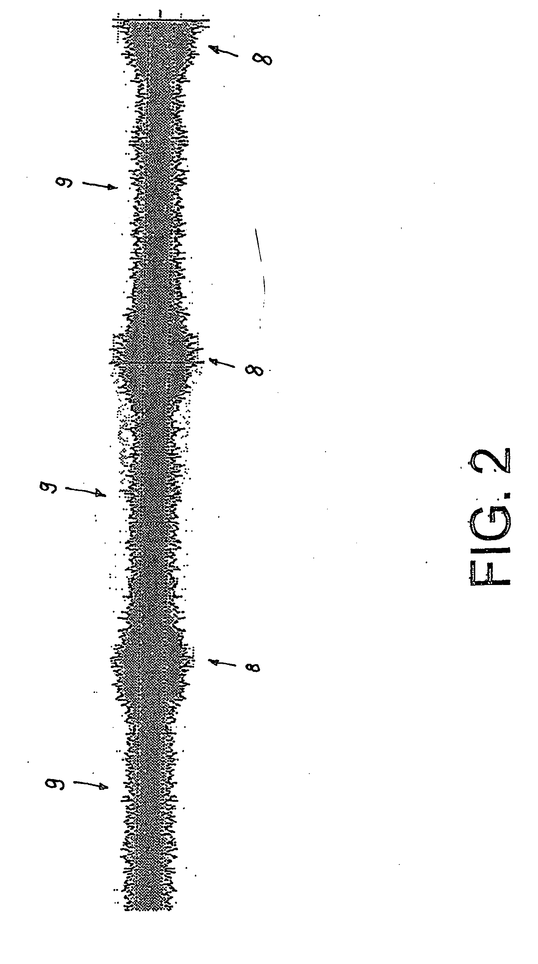

Fig. 2 zeigt die Darstellung des Effektgarnes 1 als Aneinanderreihung von Messwerten. Effekte 8 und Stege 9 sind zwar erkennbar, doch sind Beginn und Ende der Effekte 8 sowie die Effektdicke bzw. der Effektdurchmesser D_{E} und die Stegdicke bzw. der Stegdurchmesser D_{ST}, nicht eindeutig und damit nicht ausreichend erkennbar.

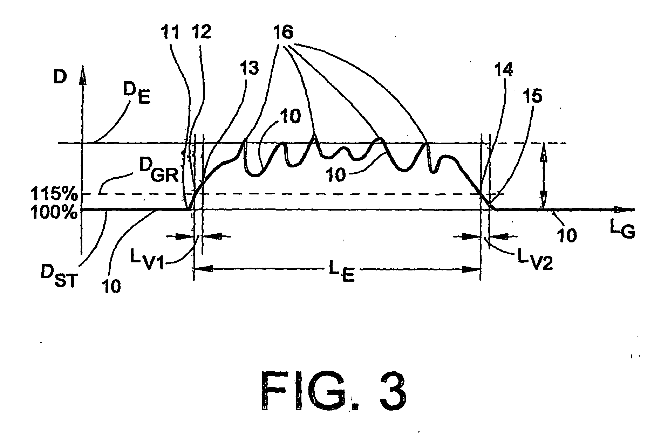

Die Auswerteeinheit 4 registriert den Garndurchmesser D jeweils nach 2 mm Garnlänge. Ein Takt repräsentiert eine Messlänge von 2 mm Garn. In der Darstellung der Fig. 3 ist der Garndurchmesser D in Prozent über die Garnlänge L_{G} als Kurve 10 dargestellt. Die Kurve 10 repräsentiert in der Darstellung der Fig. 3 von links beginnend bis zum Punkt 11 den Stegdurchmesser D_{ST}. Ab dem Punkt 11 steigt die Kurve 10 an und passiert am Punkt 12 den Wert des Grenzdurchmessers D_{GR}. Am Punkt 13 ist die vorbestimmte Garnlänge L_{V1} seit Erreichen des Punktes 12 durchgelaufen. Nachdem am Punkt 12 eine Durchmesserzunahme von 15 % registriert wird und die Überschreitung des Grenzdurchmessers D_{GR} über die vorbestimmte Länge L_{V1} z.B. sechs Takte bzw. 12 mm lang anhält, wird der Punkt 12 als Beginn des Effektes definiert. Die Kurve 10 unterschreitet den Grenzdurchmesser D_{GR} am Punkt 14. Die Unterschreitung hält bis zum Punkt 15 und somit über die vorbestimmte Garnlänge L_{V2} an. Damit wird der Punkt 14 als Ende des Effektes definiert. Aus Beginn und Ende des Effektes zwischen Punkt 12 und Punkt 14 wird die Effektlänge L_{E} ermittelt. Aus den vier größten Durchmessern 16 innerhalb des Effektes wird ein arithmetischer Mittelwert gebildet. Dadurch ist die Angabe des Effektdurchmessers weitestgehend unabhängig von natürlichen Duchmesserschwankungen im Effektbereich. Als Effektdurchmesser D_{E} wird dieser arithmetische Mittelwert definiert.

Auf der Basis der in den Fig. 2 und 3 erkennbaren Schwankungen des Garndurchmessers D im Bereich der Effektlänge der Effekte 8 wird ein Variationswert bestimmt, der eine Aussage über die Qualität der Effekte 8 ermöglicht. Der Variationswert gibt die mittlere quadratische Ungleichmäßigkeit an und ist ein Maß für die Gleichmäßigkeit des Effektverlaufes. Je höher die Gleichmäßigkeit des Effektverlaufes ist, desto besser ist die Qualität des Effektgarns 1 und des daraus hergestellten Endproduktes, beispielsweise eines Gewebes. Der Variationswert stellt die relative Streuung der Einzelwerte um den Mittelwert des Garndurchmessers D innerhalb der Effektlänge dar.

Weitere Ausbildungen des Verfahrens im Rahmen der Erfindung sind möglich. Das erfindungsgemäße Verfahren ist nicht auf das dargestellte Ausführungsbeispiel beschränkt, es wird von den nachfolgenden Ansprüchen definiert.

## Patentansprüche

1. Verfahren zur Bestimmung von Effekten eines Effektgarnes durch Messen des Garndurchmessers, wobei die Garnabschnitte zwischen den Effektbereichen als Stege bezeichnet werden,
**dadurch gekennzeichnet,**
**dass** der Effektbereich **dadurch** bestimmt wird, dass der Beginn des Effektes durch Erfüllen eines ersten Durchmesserkriteriums und dass das Ende des Effektes durch Erfüllen eines zweiten Durchmesserkriteriums definiert wird, dass zwischen Beginn und Ende des Effektes eine festlegbare Anzahl größter Durchmesser ermittelt wird,
**dass** aus diesen ermittelten größten Durchmessern ein Mittelwert gebildet wird, der als Durchmesser des Effektes festgelegt wird,
und **dass** aus Beginn und Ende des Effektes die Effektlänge bestimmt wird.

2. Verfahren nach Anspruch 1, **dadurch gekennzeichnet, daß** der Stegdurchmesser D_{ST} ermittelt wird, um die relative Effektdicke zu bestimmen.

3. Verfahren nach Anspruch 1 oder 2, **dadurch gekennzeichnet, daß** zur Bestimmung des Stegdurchmessers D_{ST} zunächst ein arithmetischer Mittelwert des Garndurchmessers aus einer vorbestimmten Länge Garn als Referenzdurchmesser gebildet wird,
daß der Referenzdurchmesser von den Einzelwerten des Garndurchmessers subtrahiert wird,
und daß dann der Stegdurchmesser D_{ST} als arithmetischer Mittelwert aus allen negativen Werten gebildet wird, die benachbart zu anderen negativen Werten gemessen wurden.

4. Verfahren nach einem der Ansprüche 1, 2 oder 3, **dadurch gekennzeichnet, dass** der Durchmesser D_{E} des Effektes als Mittelwert aus den vier größten Durchmessern zwischen Beginn und Ende des Effektes gebildet wird.

5. Verfahren nach einem der Ansprüche 1 bis 4, **dadurch gekennzeichnet, dass** als erstes Kriterium das Überschreiten eines Grenzdurchmessers D_{GR} gilt, der um einen definierten Betrag größer ist als der Stegdurchmesser D_{ST} und dass das Überschreiten über eine vorbestimmte Garnlänge L_{V1} andauert und dass als zweites Kriterium das Unterschreiten des Grenzdurchmessers D_{GR} gilt und das Unterschreiten über eine vorbestimmte Garnlänge L_{V2} andauert.

6. Verfahren nach Anspruch 5, **dadurch gekennzeichnet, dass** der Grenzdurchmesser D_{GR} 15 % größer ist als der Stegdurchmesser D_{ST}.

7. Verfahren nach Anspruch 5 oder 6 **dadurch gekennzeichnet, dass** die vorbestimmte Garnlänge dann als erreicht angenommen wird, wenn das Kriterium über sechs aufeinander folgende Messwerte erfüllt wird.

8. Verfahren nach einem der Ansprüche 1 bis 7, **dadurch gekennzeichnet, dass** beim Messen des Garndurchmessers alle zwei Millimeter ein Messwert erfasst wird.

9. Verfahren nach einem der Ansprüche 1 bis 8, **dadurch gekennzeichnet, dass** die Variation des Durchmessers auf der Effektlänge bestimmt wird.

## Claims

1. Method for determining the effects of fancy yarn by measuring the yarn diameter, whereby the yarn sections between the effect areas are referred to as webs,
**characterised in that**
the effect area is determined **in that** the beginning of the effect is defined by meeting a first diameter criterion and the end of the effect is defined by meeting a second diameter criterion, **in that** between the beginning and the end of the effect a determinable figure is determined for the greatest diameter,
**in that** from these established greatest diameters an average value is calculated which is defined as the diameter of the effect,
and **in that** the length of the effect is determined from the beginning and end of the effect.

2. Method according to claim 1, **characterised in that** the web diameter D_{ST} is established in order to determine the relative effect thickness.

3. Method according to claim 1 or 2, **characterised in that** to determine the web diameter D_{ST} firstly an arithmetic average value of the yarn diameter is formed from a predetermined length of the yarn as a reference diameter,
**in that** the reference diameter is subtracted from the individual values of the yarn diameter,
and **in that** then the web diameter D_{ST} is formed as an arithmetic average value from all of the negative values which have been measured as close to the other negative values.

4. Method according to one of claims 1, 2, or 3, **characterised in that** the diameter D_{E} of the effect is formed as an average value from the four greatest diameters between the beginning and end of the effect.

5. Method according to one of claims 1 to 4, **characterised in that** exceeding a limit diameter D_{GR} applies as a first criterion, which is greater by a defined amount than the web diameter D_{ST}, and **in that** exceeding continues over a predetermined yarn length L_{V1}, and **in that** falling below the limit diameter D_{GR} applies as a second criterion and falling below continues over a predetermined yarn length L_{V2}.

6. Method according to claim 5, **characterised in that** the limit diameter D_{GR} is 15% greater than the web diameter D_{ST}.

7. Method according to claim 5 or 6, **characterised in that** the predetermined yarn length can then be assumed to be achieved when the criterion has been met over six consecutive measured values.

8. Method according to one of claims 1 to 7, **characterised in that** on measuring the yarn diameter every two millimetres a measured value is established.

9. Method according to one of claims 1 to 8, **characterised in that** the variation of the diameter on the effect length is determined.

## Revendications

1. Procédé pour déterminer les effets d'un fil d'effet en mesurant le diamètre du fil, sachant que les tronçons de fil situés entre les zones d'effet sont appelés âmes, **caractérisé en ce que** la zone d'effet est déterminée par le fait que le début de l'effet est défini en remplissant un premier critère de diamètre et **en ce que** la fin de l'effet est défini en remplissant un deuxième critère de diamètre, **en ce qu'**on relève un nombre spécifiable de plus grands diamètres entre le début et la fin de l'effet, **en ce qu'**on forme à partir de ces plus grands diamètres relevés une valeur moyenne qui est spécifiée comme étant le diamètre de l'effet, et **en ce qu'**on détermine la longueur d'effet à partir du début et de la fin de l'effet.

2. Procédé selon la revendication 1, **caractérisé en ce qu'**on relève le diamètre d'âme D_{ST} afin de déterminer l'épaisseur d'effet relative.

3. Procédé selon la revendication 1 ou 2, **caractérisé en ce qu'**afin de déterminer le diamètre d'âme D_{ST}, on forme d'abord comme diamètre de référence une moyenne arithmétique du diamètre du fil sur une longueur prédéterminée de fil, **en ce qu'**on soustrait le diamètre de référence des valeurs individuelles du diamètre du fil, et on ce qu'on forme alors le diamètre d'âme D_{ST} comme moyenne arithmétique de toutes les valeurs négatives qui ont été mesurées au voisinage d'autres valeurs négatives.

4. Procédé selon l'une quelconque des revendications 1, 2 ou 3, **caractérisé en ce que** le diamètre D_{E} de l'effet est formé comme valeur moyenne des quatre plus grands diamètres entre le début et la fin de l'effet.

5. Procédé selon l'une quelconque des revendications 1 à 4, **caractérisé en ce que** le premier critère est le dépassement d'un diamètre limite D_{GR} qui est supérieur d'un montant défini au diamètre d'âme D_{ST}, sachant que le dépassement persiste sur une longueur prédéterminée de fil L_{V1}, et **en ce que** le deuxième critère est le sous-dépassement du diamètre limite D_{GR}, sachant que le sous-dépassement persiste sur une longueur prédéterminée de fil L_{V2}.

6. Procédé selon la revendication 5, **caractérisé en ce que** le diamètre limite D_{GR} est supérieur de 15 % au diamètre d'âme D_{ST}.

7. Procédé selon la revendication 5 ou 6, **caractérisé en ce que** la longueur prédéterminée de fil est supposée atteinte lorsque le critère est rempli pour six valeurs de mesure successives.

8. Procédé selon l'une quelconque des revendications 1 à 7, **caractérisé en ce que**, lors de la mesure du diamètre du fil, on enregistre une valeur de mesure tous les deux millimètres.

9. Procédé selon l'une quelconque des revendications 1 à 8, **caractérisé en ce qu'**on détermine la variation du diamètre sur la longueur d'effet.
